# EUROPÄISCHE PATENTSCHRIFT

(11) **EP 1 565 423 B1**
(45) Veröffentlichungstag und Bekanntmachung des Hinweises auf die Patenterteilung: **07.06.2006**
(21) Anmeldenummer: 03779986.3
(22) Anmeldetag: 19.11.2003
(51) Int. Cl.: C07C 61/04, A61K 8/36, C11B 9/00, A23L 1/22

(54) **2-HEPTYLCYCLOPROPYL-1-CARBONSÄURE**
2-HEPTYLCYCLOPROPYL-1-CARBOXYLIC ACID
ACIDE 2-HEPTYLCYCLOPROPYL-1-CARBOXYLIQUE

(30) Priorität: 21.11.2002 DE 10254265
(43) Veröffentlichungstag der Anmeldung: 24.08.2005
(73) Patentinhaber: Symrise GmbH & Co. KG, 37603 Holzminden (DE)
(72) Erfinder: WIDDER, Sabine, 37603 Holzminden (DE); LOOFT, Jan, 37603 Holzminden (DE); VAN DER KOLK, Armin, 53804 Much (DE); VÖSSING, Tobias, 37688 Beverungen (DE); PICKENHAGEN, Wilhelm, 1290 Chavannes des Bois (CH); KOHLENBERG, Birgit, 37619 Pegestorf (DE)
(74) Vertreter: Eisenführ, Speiser & Partner
(86) Internationale Anmeldenummer: PCT/EP2003/012927
(87) Internationale Veröffentlichungsnummer: WO 2004/046079

(56) Entgegenhaltungen:
- US-A- 3 926 860

## Beschreibung

Die Erfindung betrifft die neue Substanz 2-Heptylcyclopropyl-1-carbonsäure, ihre gegebenenfalls isolierten und/oder aufgereinigten Enantiomeren sowie Gemische von zwei, drei oder sämtlichen Enantiomeren der 2-Heptylcyclopropyl-1-carbonsäure.

Die Erfindung betrifft ferner Duftstoff- und/oder Aromastoffkompositionen, aromatisierte Lebensmittel, Körperpflegeprodukte, Reinigungsmittel oder sonstige nicht zum Verzehr bestimmte Produkte, die eine sensorisch wirksame Menge mindestens eines Enantiomeren der 2-Heptylcyclopropyl-1-carbonsäure enthalten.

Die Erfindung betrifft auch Verfahren zum Erzeugen oder Modifizieren von Duftstoff- oder Aromastoffkompositionen unter Verwendung einer sensorisch wirksamen Menge der 2-Heptylcyclopropyl-1-carbonsäure sowie Verfahren zur Herstellung der *cis-* und trans-Isomeren der 2-Heptylcyclopropyl-1-carbonsäure.

In der parfümistischen und flavoristischen Praxis besteht generell ein beständiger Bedarf an synthetischen Duft- und Aromastoffen, die sich günstig und mit gleichbleibender Qualität herstellen lassen, bei längerer Lagerung möglichst auch im Kontakt mit anderen Stoffen stabil bleiben und erwünschte olfaktorische bzw. geschmackliche Eigenschaften haben. Duftstoffe sollen angenehme, möglichst naturnahe Duftnoten von ausreichender Intensität aufweisen und in der Lage sein, den Duft von kosmetischen oder technischen Konsumgütern vorteilhaft zu beeinflussen. Aromastoffe sollen gut verträglich sein, an typische Geschmackskomponenten beliebter Speisen erinnern oder sogar mit diesen identisch sein und dazu beitragen den Geschmack von Lebensmitteln, oral zu verabreichenden Medikamenten und dergleichen positiv zu beeinflussen.

US-A-3 926 860 offenbart 2-Pentylcyclopropyl-1-carbonsäure sowie deren Verwendung als Duftstoff.

Das Auffinden von Duft- und Aromastoffen, die diesen Anforderungen entsprechen, hat sich als verhältnismäßig aufwendig erwiesen und erfordert regelmäßig umfangreiche Untersuchungen, insbesondere wenn interessante neuartige Duftnoten oder Geschmacksrichtungen angestrebt werden.

Die Suche nach geeigneten Duft - und Aromastoffen wird für den Fachmann insbesondere durch folgende Sachverhalte erschwert:
- Die Mechanismen der Duft- bzw. Aromawahrnehmung sind nicht ausreichend bekannt
- Eine objektive Charakterisierung eines Duftes oder Aromas ist nicht möglich
- Die Zusammenhänge zwischen der Duft- und/ oder Aromawahrnehmung einerseits und der chemischen Struktur des Duft- und/oder Aromastoffes andererseits sind nicht hinreichend erforscht.

Der Erfolg der Suche nach geeigneten Duft- oder Aromastoffen hängt deshalb in erheblichem Maße von der Intuition des Suchenden ab.

Der vorliegenden Erfindung lag zunächst die Aufgabe zugrunde, unter Beachtung der vorstehend beschriebenen generellen Rahmenbedingungen neue interessante Duft- und/oder Aromastoffe anzugeben. Die anzugebenden Substanzen sollten dem Parfümeur oder Flavoristen eine vielseitig einsetzbare Alternative zu den bislang eingesetzten oder beschriebenen Duft- und Aromastoffen bieten und insbesondere dazu geeignet sein, den sensorischen Gesamteindruck von Duftstoff- und/oder Aromastoffkompositionen, aromatisierten Lebensmitteln oder nicht zum Verzehr bestimmten Produkten positiv zu beeinflussen.

Erfindungsgemäß wird diese Aufgabe durch ein gegebenenfalls isoliertes und/oder aufgereinigtes Enantiomer der 2-Heptylcyclopropyl-1-carbonsäure oder durch ein Gemisch von zwei, drei oder sämtlichen Enantiomeren der 2-Heptylcyclopropyl-1-carbonsäure gelöst.

Die erfindungsgemäße 2-Heptylcyclopropyl-1-carbonsäure wurde nun in Orangen gefunden und aus diesen isoliert. Über sehr aufwendige analytische und präparative Verfahren wurde auf diese Weise erstmalig die 2-Heptylcyclopropyl-1-carbonsäure isoliert und identifiziert. Insgesamt ergab sich ein Gehalt von 2-Heptylcyclopropyl-1-carbonsäure im Orangen Residue von etwa 1 ppb, welches aus nur einem Enantiomer bestand. Die (1*S*, 2*R*) 2-Heptylcyclopropyl-1-carbonsäure (Formel C) wurde als die natürlich vorkommende Verbindung identifiziert.

Ausgegangen wurde bei der Isolierung von Orange Residue, einem zähflüssigen Destillationsrückstand, der aus Orange Peel Oil (Schalenöl, dieses entspricht dem Ölanteil, der sich in den Oil-Gands der Citrusschale befindet) gewonnen wird. Peel Oils sind wichtige natürliche Ausgangsmaterialien in der Aromenkreation, z.B. für Getränke oder die Citrus-Aromatisierung von Lebensmitteln. Der Anteil an Residue in einem Peel Oil liegt meist im Bereich von wenigen Massenprozent, typischerweise im Bereich von 1 bis 5 Gew.-%.

In Formel A ist die 2-Heptylcyclopropyl-1-carbonsäure ohne Angaben zur absoluten Stereochemie dargestellt: 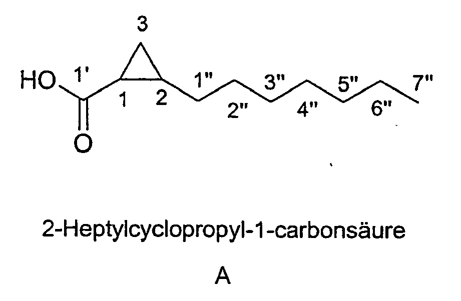 2-Heptylcyclopropyl-1-carbonsäure umfasst das Isomere *cis-2-*Heptylcyclopropyl-1-carbonsäure mit seinem Enantiomerenpaar aus (1*R*, 2*S*)-2-Heptylcyclopropyl-1-carbonsäure (dargestellt in Formel B) und (1*S*, 2*R*)-2-Heptylcyclopropyl-1-carbonsäure (dargestellt in Formel C) 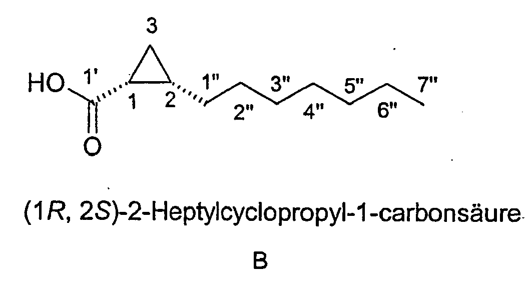 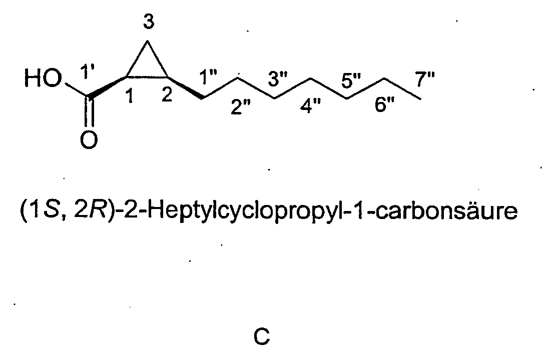 und das Isomere *trans*-2-Heptylcyclopropyl-1-carbonsäure mit seinem Enantiomerenpaar (1*R*, 2*R*)-2-Heptylcyclopropyl-1-carbonsäure (dargestellt in Formel D) und (1*S*, 2*S*)-2-Heptylcyclopropyl-1-carbonsäure (dargestellt in Formel E).

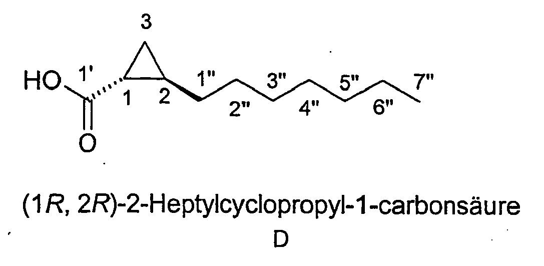

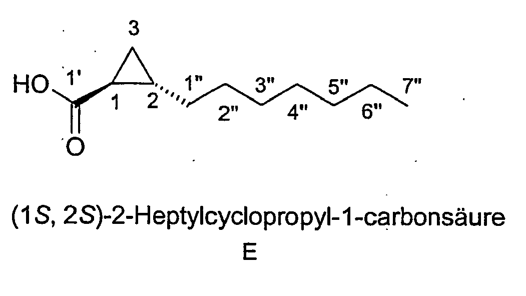

Die *cis-* und *tran*s-Isomeren sind also Diastereomere, die jeweils ein Enantiomerenpaar der 2-Heptylcyclopropyl-1-carbonsäure umfassen.

Überraschenderweise hat sich gezeigt, dass die (insgesamt vier) Enantiomeren der 2-Heptylcyclopropyl-1-carbonsäure eine sehr interessante Aromanote und/oder einen sehr interessanten Geruch besitzen. Die Enantiomeren des *cis*-Isomeren vermitteln geruchliche Noten, die sich wie folgt beschreiben lassen: holzig, balsamisch, weihrauchartig, grün, krautig, albedoartig, wachsig; die des *trans-*Isomeren vermitteln geruchliche Noten, die sich als wachsig, fettig, süß beschreiben lassen.

Gemische von zwei, drei oder allen Enantiomeren der 2-Heptylcyclopropyl-1-carbonsäure betonen oder modifizieren je nach Zusammensetzung einzelne Aspekte der individuellen sensorischen Eigenschaften der Enantiomeren. Der Fachmann wird je nach gewünschtem sensorischen Eindruck die Anteile der einzelnen Enantiomeren in einem Gemisch einstellen.

Aus dem Stand der Technik ergeben sich keine Hinweise auf die besonderen Eigenschaften der erfindungsgemäßen Enantiomeren und Enantiomerengemische:
Die US 3,926,860 offenbart als Duftstoff zwar *cis*-2-n-Pentylcyclopropyl-1-carbonsäure mit patschuliartigen animalischen und ledrigen Dufteigenschaften.
Die JP 10-165132 beschreibt zwar *cis*- und *trans*-2-n-Pentylcyclopropyl-1-carbonsäure mit besonderer Betonung der geschmacklichen Effekte, die sich mit diesen Substanzen erzielen lassen.

Es finden sich jedoch in keiner der genannten Schriften Hinweise auf 2-Heptylcyclopropyl-1-carbonsäure, und insbesondere keine Hinweise zu deren speziellen sensorischen Eigenschaften.

Als Duft- und/oder Aromastoff werden bevorzugt Gemische beider Enantiomeren (a) der *cis*-2-Heptylcyclopropyl-1-carbonsäure oder (b) der *trans*-2-Heptylcyclopropyl-1-carbonsäure eingesetzt, wobei im Gemisch (a) vorzugsweise wenig oder keine *trans*-2-Heptylcyclopropyl-1-carbonsäure (Verhältnis trans : cis < 1 : 10) und im Gemisch (b) vorzugsweise wenig oder keine *cis*-2-Heptylcyclopropyl-1-carbonsäure (Verhältnis cis : trans < 1 : 10) enthalten ist, um den sensorischen Eindruck möglichst klar in Erscheinung treten zu lassen.

Die Gemische der Enantiomeren der *trans*-2-Heptylcyclopropyl-1-carbonsäure, insbesondere das racemische Gemisch, besitzen eine wachsige, fettige, süße Duftnote, deren einzelne Aspekte sich durch entsprechende Verschiebung des Enantiomerenverhältnisses besonders betonen oder modifizieren lassen. Der Geruchsschwellenwert für das racemische Gemisch von *trans*-2-Heptylcyclopropyl-1-carbonsäure beträgt in Wasser 100 ppb (100 µg/l Wasser).

Gemische der Enantiomeren der *cis*-2-Heptylcyclopropyl-1-carbonsäure, insbesondere das racemische Gemisch, besitzen dagegen Aspekte der Duftnoten holzig, balsamisch, weihrauchärtig, grün, krautig, albedoartig und wachsig. Auch hier lassen sich einzelne Aspekte dieser Duftnoten durch geeignete Einstellung der Verhältnisse der Enantiomeren der cis-2-Heptylcyclopropyl-1-carbonsäure betonen oder modifizieren. Der Geruchschwellenwert des racemischen Gemisches der *cis*-2-Heptylcyclopropyl-1-carbonsäure beträgt in Wasser 3 ppb (3 µg/l Wasser) und ist somit extrem niedrig. Gemische der Enantiomeren der *cis*-2-Heptylcyclopropyl-1-carbonsäure, insbesondere das racemische Gemisch eignen sich hervorragend für den Einsatz in Duftstoff- und Aromastoffformulierungen. Die Gemische verleihen Aromastoffformulierungen Körper und Fülle, und es ist ein säureverstärkender Effekt feststellbar. Darüber hinaus weisen Gemische der Enantiomeren der *cis*-Isomeren der 2-Heptylcyclopropyl-1-carbonsäure eine balsamisch schalige, jedoch nicht aldehydische Aromanote auf. Das überraschende Fehlen der aldehydischen Aromanote stellt eine interessante Besonderheit dar, da schalige Aromanoten sonst regelmäßig von einer aldehydischen begleitet werden.

Als besonders vorteilhaft für die Hervorhebung bestimmter Aromaeindrücke oder Duftnoten haben sich Gemische der jeweiligen beiden Enantiomeren der *cis-* bzw. *trans*-Isomeren erwiesen, bei denen ein Enantiomerenüberschuss von zumindest 40% eingestellt ist. Mit einem solchen Enantiomerenüberschuss ließen sich die gewünschten Effekte in besonderer Deutlichkeit erzielen.

Die vorliegende Erfindung betrifft auch Duftstoff- und/oder Aromastoffkompositionen, die eine sensorisch wirksame Menge eines gegebenenfalls isolierten und/oder aufgereinigten Enantiomeren oder eines Gemisches von Enantiomeren der 2-Heptylcyclopropyl-1-carbonsäure umfassen. Bevorzugte Gemische sind dabei wieder die von beiden Enantiomeren der *cis*-2-Heptylcyclopropyl-1-carbonsäure oder der *trans*-2-Heptylcyclopropyl-1-carbonsäure mit vorzugsweise einem Enantiomerenüberschuss von zumindest 40% eines der beiden Enantiomeren.

Eine sensorisch wirksame Menge eines Enantiomeren oder Enantiomerengemisches liegt dabei insbesondere vor, wenn ein Organismus, insbesondere der Mensch in der Lage ist, die sensorische Wirkung des betreffenden Enantiomeren oder Enantiomerengemisches über seinen Geruchs- oder Geschmackssinn wahrzunehmen.

Besonders bevorzugt sind erfindungsgemäße Duftstoff- und Aromastoffkompositionen, die einen Anteil von ≥ 0,001 Massenprozent, bevorzugt ≥ 0,01 Massenprozent, vorzugsweise ≥ 0,05 Massenprozent, wiederum bevorzugt ≥ 0,1 Massenprozent und in manchen Fällen sogar ≥ 1 Massenprozent eines erfindungsgemäßen Enantiomeren bzw. eines Gemisches von erfindungsgemäßen Enantiomeren umfassen, bezogen auf die Gesamtmasse der Duftstoff- oder Aromastoffkomposition. Ein solcher Anteil eines erfindungsgemäßen Enantiomeren oder eines erfindungsgemäßen Gemisches von Enantiomeren ist in besonders effektiver Weise dazu geeignet, den Gesamteindruck von Duftstoff- und/oder Aromastoffkompositionen positiv zu beeinflussen, vgl. dazu auch Beispiel 4 unten.

Die Erfindung betrifft auch aromatisierte Lebensmittel sowie Körperpflegeprodukte, Reinigungsmittel und sonstige nicht zum Verzehr bestimmte Produkte, die jeweils eine sensorisch wirksame Menge eines erfindungsgemäßen Enantiomeren oder eines erfindungsgemäßen Gemisches von Enantiomeren umfassen.

Bevorzugt weisen die erfindungsgemäßen aromatisierten Lebensmittel, Körperpflegeprodukte, Reinigungsmittel und sonstigen nicht zum Verzehr bestimmte Produkte jeweils einen Anteil von ≥ 0,01 Massenprozent, vorzugsweise ≥ 0,05 Massenprozent, wiederum bevorzugt ≥ 0,1 Massenprozent und in manchen Fällen sogar ≥ 1 Massenprozent der erfindungsgemäße Duftstoff- und Aromastoffkompositionen auf, bezogen auf die jeweilige Gesamtmasse der aromatisierten Lebensmittel, Körperpflegeprodukte, Reinigungsmittel und sonstigen nicht zum Verzehr bestimmten Produkte.

Der Gehalt eines erfindungsgemäßen Enantiomeren bzw. eines Gemisches von erfindungsgemäßen Enantiomeren in den aromatisierten Lebensmitteln, Körperpflegeprodukten, Reinigungsmitteln und sonstigen nicht zum Verzehr bestimmten Produkten liegt vorzugsweise im Bereich von 0,01 bis 500 ppm, besonders bevorzugt im Bereich von 0,1 bis 100 ppm.

Bevorzugte erfindungsgemäße Produkte umfassen eine Menge eines erfindungsgemäßen Enantiomeren oder eines erfindungsgemäßen Gemisches von Enantiomeren zusätzlich zu einer oder mehreren Substanzen aus der Gruppe, die besteht aus: Natürlichen Parfümölen; synthetischen Parfümölen; etherischen Ölen; natürlichen Pflanzenextrakten; Alkoholen; Aldehyden; Ketonen; Estern; Lactonen; Carbonsäuren; Detergenzien; Seifen; Badepräparaten; Haarpräparaten; Kosmetischen Präparaten; Pudern; synthetischen Kohlehydraten; natürlichen Kohlehydraten; synthetischen Fetten; natürlichen Fetten; Proteinen; Vitaminen.

Ebenfalls Bestandteil der Erfindung ist demgemäß die Verwendung eines erfindungsgemäßen Enantiomeren oder eines erfindungsgemäßen Gemisches von Enantiomeren als Duft- und/oder Aromastoff.

Die Verwendung der erfindungsgemäßen Enantiomeren und Enantiomerengemische als Duftstoffe, und zwar insbesondere als Duftstoffe in der Parfümindustrie, bietet sich ganz besonders für die *cis-*2-Heptylcyclopropyl-1-carbonsäure an, deren geruchliche Eigenschaften von Parfümeuren besonders geschätzt werden. Der niedrige Geruchsschwellenwert des *cis*-Isomeren erweist sich als vorteilhaft , da bereits geringe Mengen dieser erfindungsgemäßen Substanz zur Erzeugung eines erwünschten Geruches ausreichen. Aber auch die Enantiomeren des trans-Isomeren und deren Mischungen weisen hervorragende sensorische Eigenschaften und einen recht niedrigen Geruchsschwellenwert auf, so dass sie für die Verwendung als Duftstoff vorzüglich geeignet sind.

Ein entsprechendes erfindungsgemäßes Verfahren zum Erzeugen oder Modifizieren einer Duftstoff- oder Aromastoffkomposition enthält die folgenden Schritte:
- Bereitstellen einer Ausgangskomposition und
- Vermischen der Ausgangskomposition mit einer sensorisch wirksamen Menge eines Enantiomeren oder eines erfindungsgemäßen Gemisches von Enantiomeren.

Auf diese Weise lassen sich gewünschte Duft- und/oder Aromaeffekte erzielen, z.B. die Verschiebung eines gegebenen Aromaeindruckes hin zu frischer, fruchtiger, voller und/oder schaliger (vgl. auch Beispiel 4).

Die Erfindung betrifft auch ein Verfahren zur selektiven Herstellung von *cis*- oder *trans*-2-Heptylcyclopropyl-1-carbonsäure, wobei zur Einstellung der *cis*- bzw. *trans*-Konfiguration der Substituenten am Cyclopropan-Ring von einem Precursor mit einer Z- bzw. E-konfigurierten Doppelbindung ausgegangen und diese Doppelbindung selektiv cyclopropaniert wird. Mit dem genannten Verfahren lässt sich mit hoher Selektivität die erwünschte *cis*- bzw. *trans*-2-Heptylcyclopropyl-1-carbonsäure darstellen.

Die Erfindung betrifft ferner Verfahren zur Herstellung von *cis-2-*Heptylcyclopropyl-1-carbonsäure, bei denen (a) nach Kettenverlängerung von Propargylalkohol 2-Decin-1-ol erhalten wird, welches anschließend stereoselektiv hydriert und stereoselektiv cyclopropaniert wird und/oder (b) *cis*-2-Heptylcyclopropyl-1-methanol zu *cis*-2-Heptylcyclopropyl-1-carbonsäure oxidiert wird. Die Cyclopropanierung kann gegebenenfalls unter Verwendung chiraler Hilfsstoffe enantioselektiv erfolgen. Zur Oxidation des Heptylcyclopropyl-1-methanols zur *cis*-2-Heptylcyclopropyl-1-carbonsäure können z.B. Chromreagenzien wie Chromschwefelsäure, aber auch Verfahren, die sich z.B. des freien Radikals TEMPO bedienen, verwendet werden.

Ebenfalls Teil der Erfindung sind Verfahren, bei denen nach Wittig-Horner-Reaktion an Octanal mit nachfolgender trans-selektiver Cyclopropanierung des erhaltenen 2-(E)-Decensäureethylesters durch anschließende basische Esterhydrolyse *trans-*2-Heptylcyclopropyl-1-carbonsäure hergestellt wird.

Die Erfindung betrifft weiterhin Verfahren zur Herstellung von 2-Heptylcyclopropyl-1-carbonsäure, bei denen die *cis-* und *trans*-Verbindungen als Gemisch erhalten werden. Die Verfahren umfassen z.B.
(a) Die Übergangsmetall-katalysierte Reaktion von 1-Nonen mit Diazoessigester. Hierbei können achirale Verbindungen wie Rhodiumacetat oder Kupferbronze zum Einsatz kommen, aber auch Übergangsmetallkomplexe mit chiralen Liganden wie z.B. Bisoxazoline oder Schiffsche Basen sind geeignet. Die Zusammensetzung der so erhaltenen *cis-*/*trans-*2-Heptylcyclopropyl-1-carbonsäureestergemische kann durch Destillation eingestellt werden. Die *cis*-/*trans*-2-Heptylcyclopropyl-1-carbonsäureestergemische werden zur 2-Heptylcyclopropyl-1-carbonsäure verseift. Da unter geeigneten Solvolyse-Bedingungen (z.B. wässrige KOH oder NaOH in wässriger ethanolischer Lösung) der *trans*-2-Heptylcyclopropyl-1-carbonsäureester schneller verseift, kann auf diese Weise der *cis*-/*trans*-Gehalt ebenfalls eingestellt werden.
(b) Die Reaktion von 1,3-Dicarboxylverbindungen wie z.B. Malonaten oder Meldrumsäure mit 1,2-disubstituierten Nonanen wie z.B. 1,2-Dibromnonan oder 4-Heptyl-[1,3,2]dioxathiolan-2,2-dioxid. Durch die Wahl geeigneter chiraler 1,2-disubstituierten Nonane können die 2-Heptylcyclopropyl-1-dicarbonsäureester in enantiomerenangereicherter Form erhalten werden. Die 2-Heptylcyclopropyl-1-dicarbonsäureester können entweder direkt decarboxyliert und anschließend verseift oder aber zunächst verseift und die erhaltene 2-Heptylcyclopropyl-1-dicarbonsäure anschließend decarboxyliert werden. Ebenfalls möglich ist die Decarboxylierung der entsprechenden Monoester oder von gemischten Estern.

Die Zusammensetztung der durch diese Verfahren hergestellten *cis-*/trans-2-Heptylcyclopropyl-1-carbonsäuregemische läßt sich durch Destillation einstellen.

Ein weiterer Bestandteil der Erfindung ist es, in Gemischen, die mehr als ein Enantiomer der 2-Heptylcyclopropyl-1-carbonsäure enthalten, bevorzugt in Gemischen der jeweiligen Enantiomeren der *cis*- oder der trans-2-Heptylcyclopropyl-1-carbonsäure einzelne Enantiomeren anzureichern und/oder die Enantiomeren eines solchen voneinander zu trennen. Der Fachmann wird dazu geeignete Wege wählen, wie beispielsweise die Trennung mittels chiraler Gaschromatographie.

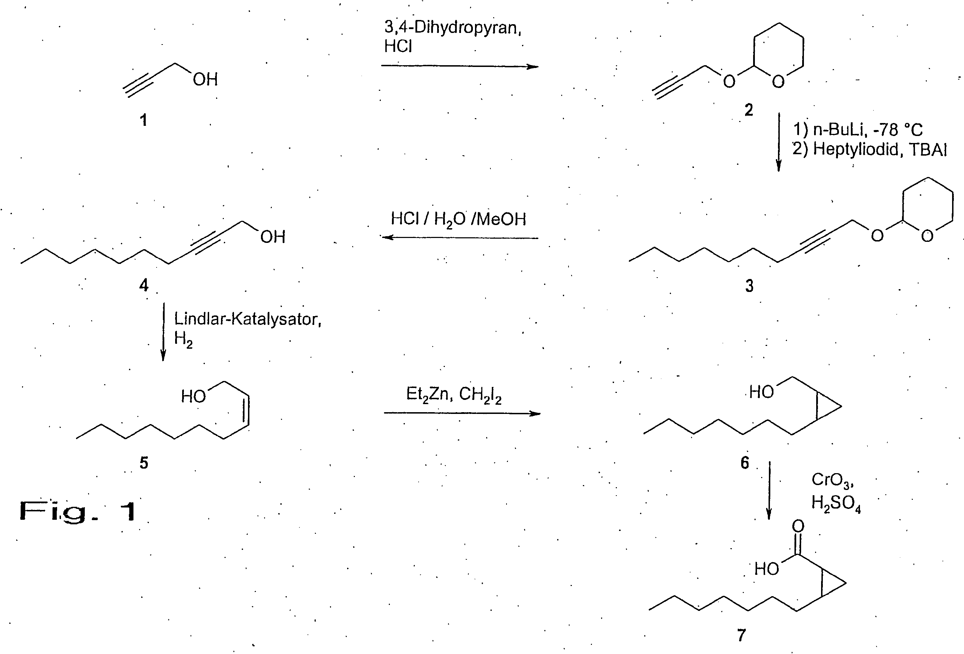

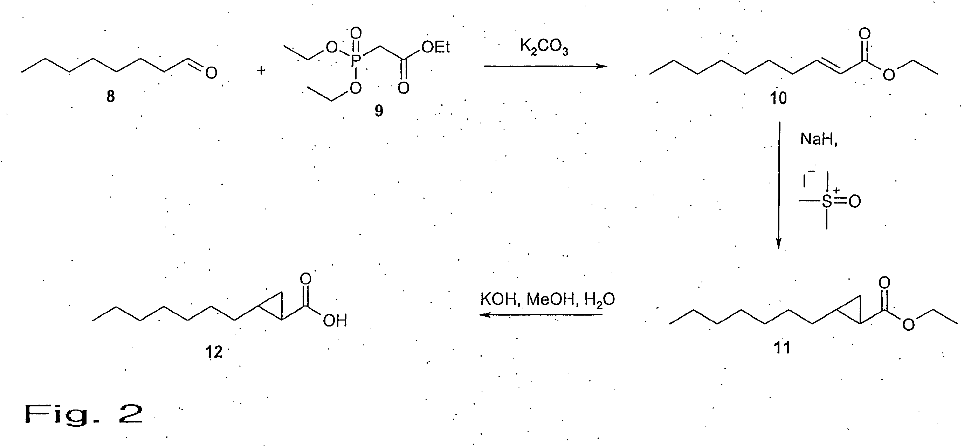

Die Erfindung wird nachfolgend anhand von Beispielen unter Bezugnahme auf die beigefügten Figuren erläutert. Dabei entsprechen die Zahlen in Klammern hinter den Substanznamen den Zahlen in den Figuren 1 und 2.

### Beispiel 1: Synthese von cis-2-Heptylcyclopropyl-1-carbonsäure (7)

Die Synthese ist in Figur 1 schematisch dargestellt.

Propargylalkohol (2-Propin-1-ol) wird als THP-Ether (TetrahydropyranylEther) geschützt, mit *n*-BuLi lithiiert und mit Heptyliodid um eine C₇₋Einheit verlängert. Das 2-Decin-1-ol wird durch Umacetalisierung auf Methanol entschützt. Die *cis*-Konfiguration der Substituenten am Cyclopropanring von *cis*-2-Heptylcyclopropyl-1-methanol wird durch die syn-selektive (cis-selektive) Hydrierung am Lindlar-Katalysator und nachfolgende syn-selektive (cis-selektive) Cyclopropanierung mit einer modifizierten Methode nach Simmons-Smith gewährleistet. Selektivität der Reaktionen: Hydrierung an Lindlar-Katalysator: E. N. Marvell, T. Li, *Synthesis* **1973,** 457- 468; Simmons-Smith-Reaktion: H. E. Simmons, E. P. Blanchard, R. D. Smith, *J*. *Am. Chem. Soc.* **1964,** *86,* 1347-1356). Anschließend erfolgt die Oxidation zur *cis*-2-Heptylcyclopropyl-1-carbonsäure mit Chrom-Schwefelsäure.

### 2-Propinyltetrahydro-2H-pyran-2-ylether (2)

Zu 25.2 g (0.3 mol) 3,4-Dihydropyran wurde bei 5 °C unter Rühren 1 ml HCl (32 %ig) gegeben. Innerhalb von 30 min wurden 11.2 g (11.8 ml, 0.2 mol) 2-Propin-1-ol (1) so zudosiert, dass 10 °C nicht überschritten wurden. Man rührte 2 h bei 5 °C und 18 h bei Zimmertemperatur. Man setzte 100 ml Diethylether zu und wusch zweimal mit je 20 ml gesättigter Natriumhydrogencarbonatlösung und 20 ml gesättigter Kochsalzlösung. Man trocknete über Natriumsulfat und destillierte den Ether am Rotationsverdampfer ab. Das Rohprodukt wurde an 300.g Kieselgel 60 (230-400 mesh.) (Hexan/ Diethylether= 9 /1) chromatographiert.

Es wurden 12.3 g 2-Propintetrahydro-2H-pyran-2-ylether (GC: >95 % Reinheit) erhalten.

Durchführung in Anlehnung an: L. F. Tietze, Th. Eicher, *Reaktionen und Synthesen,* Georg Thieme Verlag Stuttgart, New York, 2. Auflage 1991, S. 211 - 212.

¹H-NMR (400 MHz, CDCl₃): δ = 4.81 (t, *J =* 3.3 Hz, 1 H), 4.26 (dd, *J =* 2.4 Hz und 24.7 Hz, 1 H), 4.22 (dd, *J* = 2.4 Hz und 24.7 Hz, 1 H), 3.89 - 3.77 (m, 1 H), 3.59 - 3.47 (m, 1 H), 2.43 (t, *J* = 2.4 Hz, 1 H), 1.83 - 1.47 (m, 6H).

s: Singulett, d: Dublett, t: Triplett, q: Quartett, quin: Quintett, m: Multiplett, S_{b}: Singulett breit, m_{c}: Multiplett zentriert, OH: Hydroxygruppe.

¹³C-NMR (100 MHz. CDCl₃): δ = 96.52 (CH), 79.63 (C), 73.90 (CH), 61.74, 53.81, 30.17, 25.34, 18.98 (CH₂).

Signalmultiplizitäten durch DEPT-Experimente bestimmt.

FTIR (gas phase): 3328 (w), 2950 (m), 2879 (w), 1452 (w), 1350 (w), 1261 (w), 1202 (w), 1128 (m), 1038 (s), 954 (w), 904 (w), 874 (w), 816 (w).

s: strong, m: middle, w: weak.

MS (EI, 70 eV): 139 (M⁺, 9), 101 (6), 85 (100), 82 (12), 67 (11), 56 (49), 41 (51), 39 (56), 29 (34).

### 2-Decinyltetrahydro-2H-pyran-2-ylether (3)

Zu einer Lösung aus 35.01 g 2-Propintetrahydro-2H-pyran-2-ylether (0.25 mol) in 600 ml trockenem Tetrahydrofuran (THF) wurden unter Schutzgas und Rühren bei -80 °C 100 ml n-BuLi (2.5 M in Hexan) so zugetropft, dass -78 °C nicht überschritten wurden. Man ließ die Temperatur über 45 min auf Zimmertemperatur steigen, fügte 9.3 g Tetrabutylammoniumiodid (TBAI) (25 mmol) und 56.5 g 1-Heptyliodid (0.25 mol) zu und erhitzte 16 h unter Rückfluss.

Die Reaktion wurde durch vorsichtige Zugabe von 100 ml Wasser beendet. Man verdünnte mit 500 ml Diethylether, trennte die organische Phase ab, wusch mit gesättigter Kochsalzlösung und trocknete über Natriumsulfat. Die organischen Lösungsmittel wurden am Rotationsverdampfer entfernt und das Rohprodukt im Kugelrohr destilliert.

Man erhielt 38,5 g eines Gemisches aus 19 % 1-Heptyliodid, 26 % Decinol und 55 % 2-Decinyltetrahydro-2*H*-pyran-2-ylether. Dieses Rohprodukt wurde ohne weitere Aufreinigung für die Entschützung zum 2-Decin-1-ol verwendet.

Durchführung analog zu: M. Buck, J. M. Chong, *Tetrahedron Letters* **2001,** *42*, 5825-5827.

MS (EI, 70 eV): 237 (M⁺, 0.5), 167 (2), 111 (11), 101 (32), 95 (46), 85 (100), 81 (51), 67 (48), 55 (48), 41 (50).

### 2-Decin-1-ol (4)

Der rohe 2-Decinyltetrahydro-2*H*-pyran-2-ylether wurde mit 100 ml Wasser, 100 ml THF, 50 ml Methanol und 2 ml Salzsäure (4 N) unter Rückfluß erhitzt. Man verdünnte mit 300 ml Diethylether, trennte die organische Phase ab, wusch mit gesättigter Kochsalzlösung und trocknete über Natriumsulfat. Die organischen Lösungsmittel wurden am Rotationsverdampfer entfernt und das Rohprodukt im Kugelrohr destilliert.

Man erhielt 25.2 g 2-Decin-1-ol (Reinheit nach GC: 85%; Sdp. = ca. 120 °C bei 5 mbar).

Durchführung in Anlehnung an: L. F. Tietze, Th. Eicher, *Reaktionen und Synthesen,* Georg Thieme Verlag Stuttgart, New York , 2. Auflage 191, S.413.

¹H-NMR (400 MHz, CDCl₃): δ = 4.23 (s_{b}, 2 H), 2.63 (s_{b}, 1 H), 2.19 (tt, *J* = 2.2 Hz und *J* = 7.3 Hz, 2 H), 1.49 (quint, *J* = 7.3 Hz, 2 H), 1.42 -1.21 (m, 8 H), 0.89 (t, *J =* 6.8 Hz, 3 H).

¹³C-NMR (100 MHz, CDCl₃): δ = 86.17 (C), 78.27 (C), 51.05, 31.73, 28.86, 28.82, 28.66, 22.64, 18.75 (CH₂), 14.06 (CH₃).

FTIR (gas phase): 3662 (w), 2938 (s), 2871 (m), 2285 (w), 2221 (w), 1460 (w), 1387 (m), 1137 (w), 1012 (m).

MS (EI, 70 eV): 153 (M⁺, 0.5), 123 (11), 111 (17), 107 (16), 93 (45), 81 (61), 70 (64), 67 (88), 55 (100), 41 (99), 29 (50).

### (Z)-2-Decen-1-ol (5)

Man rührte 20 g rohes 2-Decin-1-ol (85 %ig, 110 mmol) und 4 g Lindlar-Katalysator in 150 ml Hexan unter einer Wasserstoffatmosphäre bei Normaldruck. Nach 24 h wurde über Kieselgel 60 filtriert und das Lösungsmittel am Rotationsverdampfer entfernt.

Man erhielt 14.5 g rohes (Z)-2-Decen-1-ol mit einer Reinheit von 81.5 %. (Verunreinigungen: 10 % Heptyliodid, 8.5 % Decinol).

Durchführung in Anlehnung an: K.-K. Chan, N. Cohen, J. P. De Noble, A. C. Specian Jr., G. Saucy, *J. Org. Chem.* **1950**, *41*, 3497 - 3505.

¹H-NMR (400 MHz, CDCl₃): δ = 5.55 (m_{c}, 2 H), 4.18 (d, J = 6.2 Hz, 2 H), 2.06 (m_{c}, 2 H), 1.75 (s_{b}, 1 H), 1.43-1.20 (m, 10 H), 0.89 (t, J = 7.0 Hz, 3 H).

¹³C-N MR (100 MHz, CDCl₃): δ = 132.5 (CH), 128.3 (CH), 58.28 (CH₂), 31.83, 29.64, 29.20, 29.17, 27.44, 22.66 (CH₂), 14.08 (CH₃).

FTIR (gas Phase): 3659 (w), 3020 (w), 2934 (s), 2868 (m), -1640 (w); 1462 (w), 1371 (w), 1024 (m).

MS (EI, 70 eV): 156 (M, 0.5), 138 (10), 109 (11), 95 (18), 81 (30), 67 (36), 57 (100), 41 (59), 29 (31)

### cis-2-Heptylcyclopropyl-1-methanol (6)

Unter Argon wurden 100 ml Diethylzink-Lösung (1,0 M in Hexan) und 100 ml trockener Diethylether bei -10 °C gerührt und 6.25 g (Z)-2-Decen-1-ol (0.04 mol) in 20 ml Diethylether so zugetropft, dass die Temperatur 0°C nicht überstieg. Es wurden 8.1 ml Dilodmethan (0.1 mol) zugefügt und 2 h bei 0°C und 16 h bei Zimmertemperatur gerührt.

Man kühlte auf 0°C ab und versetzte mit 100 ml Salzsäure (4 N). Die organische Phase wurde mit 100 ml gesättigter Natriumhydrogencarbonatlösung und 50 ml gesättigter Kochsalzlösung gewaschen, über Natriumsulfat getrocknet und das Lösungsmittel am Rotationsverdampfer entfernt. Das Rohprodukt wurde bei ca. 130 °C/7 mbar im Kugelrohr destilliert. Anschließend chromatographierte man an Kieselgel 60 (Hexan/ Ether = 6/1).

Man erhielt 3.4 g cis-2-Heptyl-cyclopropyl-1-methanol von 95%iger Reinheit.

Durchführung in Anlehnung an: T. Oshima, K. Kagechika, M. Adachi, M. Sodeoka, M. Shibasaki, *J. Am. Chem. Soc*. **1996**, 118, 7108 - 7116.

¹H-NMR (400 MHz, CDCl₃): δ = 3.59 (m_{c}, 2 H), 2.26 (S_{b}, 1 H), 1.50-1.16 (m, 12 H), 1.08 (m_{c}, 1 H), 0.88 (t, *J =* 7.0 Hz, 3 H), 0.87 - 0.80 (m, 1 H), 0.69 (m_{c}, 1 H), -0.040 (m_{c}, 1 H).

¹³C-NMR (100 MHz, CDCl₃): δ = 62.96, 31.91, 30.18, 29.61, 29.34, 28.59, 22.69 (CH₂), 18.07, 16.18 (CH), 14.10 (CH₃), 9.55 (CH₂).

FTIR (gas Phase): 3656 (w), 3071 (w), 3005 (w), 2933 (s), 2868 (m), 1463 (w), 1403 (w), 1353 (w), 1030 (m).

MS (EI, 70 eV): 152 (1), 129 (14), 111 (21), 95 (12), 81 (31), 69 (100), 57 (57), 55 (76), 43 (61), 41 (80), 29 (35).

### cis-2-Heptylcyclopropyl-1-carbonsäure (7)

Man löste 2.5 g Chromtrioxid (25 mmol) in 60 ml Schwefelsäure (1 M, 60 mmol, 2.4 eq). Unter Rühren wurden innerhalb von 1 h bei 0°C 2.1 g *cis*-2-Heptyl-cyclopropyl-1-methanol in 100 ml Aceton zugetropft. Man rührte 18 h bei Zimmertemperatur.

Es wurden 300 ml Diethylether zugefügt und die Phasen getrennt. Die wässrige Phase wurde dreimal mit je 100 ml Diethylether extrahiert, die organischen Phasen vereinigt und dreimal mit je 50 ml gesättigter Kochsalzlösung gewaschen.

Die organische Säure wurde durch Waschen mit viermal je 50 ml Natronlauge (5 %ig) abgetrennt. Man wusch zweimal mit 50 ml Diethylether und setzte die Säure mit 150 ml Salzsäure (4 N) frei. Man extrahierte fünfmal mit je 100 ml Diethylether. Die organische Phase wurde mit 50 ml gesättigter Natriumchloridlösung gewaschen und über Natriumsulfat getrocknet.

Nach Eindampfen am Rotationsverdampfer wurde das Rohprodukt an Kieselgel 60 (Cyclohexan/Diethylether = 3/1) chromatographiert.

Man erhielt 0.72 g *cis*-2-Heptyl-cyclopropyl-1-carbonsäure mit einer Reinheit >98 % (GC).

Durchführung analog zu: J. G. Millar, A. C. Oehlschlager, J. W. Wong, J. *Org. Chem.* **1983**, 48, 4404 - 4407.

¹H-NMR (400 MHz, CDCl₃): δ = 11.8 (s_{b}, 1 H), 1.65 (m_{c}, 1 H), 1.55 (m_{c}, 1H), 1.48 - 1.15 (m, 12 H), 1.06 (m_{c}, 1 H), 0.96 (m_{c}, 1 H), 0.88 (t, *J* = 6.4 Hz, 3 H).

¹³C-NMR (100 MHz, CDCl₃): δ = 179.8 (COO), 31.85, 29.54, 29.28, 29.26, 26.92 (CH₂), 23.22 (CH), 22.68 (CH₂), 18.13 (CH), 14.44 (CH₂), 14.09 (CH₃).

FTIR (gas phase): 3580 (m), 3016 (w), 2967 (m), 2934 (s), 2869 (m), 1767 (s), 1458 (w), 1414 (w), 1367 (w), 1175 (w), 1133 (s), 883 (w).

MS (EI, 70 eV): 137 (6), 123 (11), 114 (11), 99 (21), 84 (36), 73 (100), 69 (41), 55 (73), 43 (48), 41 (62), 29 (32).

### Beispiel 2: Synthese von trans-2-Heptylcyclopropyl-1-carbonsäure (12)

Die Synthese ist in Figur 2 schematisch dargestellt.

Wittig-Horner-Reaktion von Octanal mit Triethylphosphonoacetat liefert (*E*)-2-Decensäureethylester. (Zur Stereoselektivität vergleiche: J. Villieras, M. Rambaud, *Synfhesis* **1983,** 300 - 303; R. Brückner, *Reaktionsmechanismen,* Spektrum Akademischer Verlag, Heidelberg, Berlin, Oxford, **1.** Auflage 1996, S. 321). Durch Cyclopropanierung mit Dimethylsulfoxoniummethylid wird selektiv *trans*-2-Heptylcyclopropyl-1-carbonsäureethylester erhalten: (Zur Stereoselektivität: Laut S. R. Landor, N. Punja, *J. Org. Chem.* 1967, 2495 - 2500 führen (E)-konfigurierte α,β-ungesättigte Ester zu *trans*-substituierten Cyclopropanen, (Z)- konfigurierte α,β-ungesättigte Ester wurden nicht untersucht. Nach R. Brückner, *Reaktionsmechanismen,* Spektrum Akademischer Verlag, Heidelberg, Berlin, Oxford, 1. Auflage 1996, S. 321 liefern sowohl (E)- als auch (Z)- konfigurierte α,β-ungesättigte Ester trans-substituierte Cyclopropane). Milde Verseifung mit Kaliumhydroxid bei Zimmertemperatur ergibt *trans*-2-Heptylcyclopropyl-1-carbonsäure.

### (E)-2-Decensäureethylester (10)

Zu 150 g Eiswasser und 62.0 g Kaliumcarbonat (0.45 mol) wurden innerhalb von 5 min 50.5 g Triethylphosphonoacetat (9) (0.225 mol), anschließend innerhalb von 30 min 19.23 g Octanal (8) (0.15 mol) zugetropft. Die Temperatur sollte 15 °C nicht überschreiten. Man rührte 20 h bei Raumtemperatur.

Es wurden 350 ml Hexan zugefügt, die organische Phase abgetrennt und zweimal mit je 150 ml Wasser und einmal mit 100 ml gesättigter Kochsalzlösung gewaschen. Man trocknet über Natriumsulfat und dampfte das Lösungsmittel am Rotationsverdampfer ab. Das Rohprodukt wurde im Kugelrohr bei 180-185 °C / 6-7 mbar destilliert.

Man erhielt 27.4 g (Z)-2-Decensäureethylester von >95 %iger Reinheit.

Literatur: J. Villieras, M. Rambaud, *Synthesis* **1983,** 300 - 303.

¹H-NMR (400 MHz. CDCl₃): δ = 6.95 (dt, *J* = 7.0 Hz und 15.8 Hz, 1 H), 5.79 (dt, J = 15.4 Hz und 1.5 Hz, 1H), 4.17 (q, J = 7.0 Hz, 2 H), 2.18 (dq, J = 1.5 Hz und 7.0 Hz, 2 H), 1.46 (m_{c}, 1 H), 1.38-1.22 (m, 12 H), 0.88 (t, 3 H).

¹³C-NMR (100 MHz, CDCl₃): δ = 166.3 (COO), 149.0 (CH), 121.1 (CH), 59.93, 32.20, 31.82, 29.14, 29.09, 28.08, 22.66 (CH₂), 14.29, 14.06 (CH₃).

FTIR (gas phase): ∼2966 (m), 2937 (s), 2868 (m), 1740 (s), 1656 (w) 1464 (w), 1370 (w), 1310 (m), 1260 (s), 1167 (s), 1124 (w), 1047 (m), 978 (w), 858 (w).

MS (EI, 70 eV): 153 (61), 127 (20), 115 (20), 110 (41), 101 (76), 88 (38), 84 (40), 73 (85), 69 (57), 55 (100), 41 (83), 29 (73).

### trans-2-Heptylcyclopropyl-1-carbonsäureethylester (11)

Unter Schutzgas wurden 0.84 g Natriumhydrid (35.0 mmol) in 25 ml trockenem Dimethylsulfoxid suspendiert und 6.60 g Trimethylsulfoxoniumiodid zugefügt. Man rührte 30 min, wobei starke Gasentwicklung und ein leichter Temperaturanstieg zu beobachten waren. Man fügte 4.96 g (*E*)-2-Decensäureethylester (25 mmol) über 15 min hinzu und rührte 150 min bei Zimmertemperatur.

Es wurden vorsichtig 100 ml Wasser zugesetzt und mit 500 ml Diethylether verdünnt. Die organische Phase wurde abgetrennt, mit gesättigter Natriumchloridlösung gewaschen, über Natriumsulfat getrocknet und der Diethylether am Rotationsverdampfer entfernt.

Das Rohprodukt wurde an Kieselgel 60, (Hexan/ Diethylether = 6/1) chromatographiert.

Man erhielt 1.1 g *trans-*2-Heptyl-cyclopropyl-1-carbonsäureethylester.

Durchführung analog zu: S. R. Landor, N. Punja, *J. Chem. Soc.* **1967**, 2495 -2500.

¹H-NMR (400 MHz, CDCl₃): δ = 4.10 (q, *J* = 7.2 Hz, 2 H), 1.45 -1.22 (m, 17 H), 1.13 (m_{c}, 1 H), 0.86 (t, 3 H, J = 7.0 Hz), 0.67 (m_{c}, 1 H).

¹³C-NMR (100 MHz, CDCl₃): δ = 174.1 (COO), 60.11, 33.07, 31.84, 29.27, 29.21, 29.11 (CH₂), 22.87 (CH), 22.66 (CH₂), 20.22 (CH), 15.45 (CH₂), 14.29, 14.08 (CH₃).

FTIR (gas Phase): 2966 (m), 2933 (s), 2865 (m), 1745 (s), 1456 (w), 1410 (w), 1371 (w), 1339 (w), 1259 (m), 1171 (s), 1083 (w), 1046 (w), 861 (w).

MS (EI, 70 eV): 212 (M, 1), 167 (23), 128 (39), 123 (14), 101 (91), 99 (33), 88 (40), 81 (32), 73 (80), 69 (52), 55 (100), 41 (65), 29 (54).

### trans-2-Heptylcyclopropyl-1-carbonsäure (12)

Man rührte 900 mg *trans*-2-Heptyl-cyclopropyl-1-carbonsäureethylester (4.24 mmol) mit 720 mg Kaliumhydroxid (12.8 mmol) in 2 ml Wasser und 3 ml Ethanol 4.5 h bei Zimmertemperatur. Mit verdünnter Salzsäure (1 M) wurde pH = 2 eingestellt und die wässrige Phase dreimal mit je 30 ml Diethylether extrahiert. Man wusch mit 20 ml gesättigter Natriumchloridlösung, trocknete über Natriumsulfat und destillierte die Lösungsmittel am Rotationsverdampfer ab.

Es wurden 780 mg trans-2-Heptyl-cyclopropyl-1-carbonsäure mit einer Reinheit >98 % (GC) erhalten.

Durchführung in Anlehnung an: L. F. Tietze, Th. Eicher, *Reaktionen und Synthesen,* Georg Thieme Verlag Stuttgart, New York , 2. Auflage 191, S.491-492.

¹H-NMR (400 MHz. CDCl₃): δ = 8.72 (S_{b}, 1 H), 1.50 - 1.17 (m, 15 H), 0,88 (t, *J* = 6.8 Hz, 3 H), 0.74 (ddd, *J* = 4.0, 6.6, 10.6 Hz, 1 H).

¹³C-NMR (100 MHz, CDCl₃): δ = 180.6 (COO), 33.01, 31.78, 29.20, 29.20, 29.02 (CH₂), 23.97 (CH), 22.63 (CH₂), 20.10 (CH), 16.32 (CH₂), 14.06 (CH₃)

FTIR (gas Phase): 3580 (m), 3016 (w), 2967 (m), 2934 (s), 2866 (m), 1767 (s), 1458 (w), 1415 (w), 1366 (w), 1175 (w), 1132 (s).

MS (EI, 70 eV): 138 (3), 124 (14), 113 (15), 99 (23), 84 (38), 73 (100), 69 (55), 55 (86), 43 (60), 41 (75), 29 (37).

### Beispiel 3: Sensorische Untersuchungen von cis-2-Heptylcyclopropyl-1-carbönsäure und trans-2-Heptylcyclopropyl-1-carbonsäure

Die Verbindung umfasst 2 Diastereomere mit jeweils 2 Enantiomeren. Die beiden racemischen Diastereomere unterscheiden sich deutliche in ihren geruchlichen Eigenschaften. Das cis-Isomere weist eine deutlich niedrigere Geruchsschwelle als das trans-Isomere auf.

### Cis-2-heptyl-cyclopropyl-1-carbonsäure (racemisch)

Geruchsbeschreibung:
holzig, balsamisch, weihrauchartig, grün, krautig, albedoartig, wachsig

Geruchsschwelle in Wasser:
3 ppb (3 µg/l Wasser)

### Trans-2-heptyl-cyclopropyl-1-carbonsäure (racemisch)

Geruchsbeschreibung:
Wachsig, fettig, süß

Geruchsschwelle in Wasser:
100 ppb (100 µg/l Wasser)

### Beispiel 4: Aromaformutierung

**Tabelle 1**

| Komponente | Menge [%] | |
|---|---|---|
| | A | B |
| Orangenöl bras. grün | 40 | 40 |
| cis-2-Heptylcyclopropyl-1-carbonsäure | - | 0,1 |
| Ethylbutyrat | 2 | 2 |
| Aldehyd C 12 | 0,03 | 0,03 |
| Aldehyd C10 | 0,05 | 0,05 |
| Linalool | 0,2 | 0,2 |
| Orangenöl Florida | 15 | 15 |
| Orange Essence Öl | 42,72 | 42,62 |
| Summe | 100 | 100 |

Eine übliche Aromenkomposition (Komposition A) wurde mit einer erfindungsgemäßen Aromenkomposition (Komposition B) verglichen. Wie sich aus obiger Tabelle 1 ergibt, entsprach Komposition B der Komposition A weitgehend, aber Komposition B enthielt im Unterschied zu Komposition A einen Anteil von 0,1 % cis-2-Heptylcyclopropyl-1-carbonsäure (bezogen auf die Gesamtmasse der Komposition), was durch eine Verringerung des Anteils an Orange Essence Öl ausgeglichen wurde.

Das Aroma der erfindungsgemäßen Komposition war im Vergleich mit dem Aroma der herkömmlichen Komposition A frischer, fruchtiger, voller und schaliger.

### Beispiel 5: Synthese von enantiomerenangereicherter cis-2-Heptylcyclopropyl-1-carbonsäure (1S, 2R)

### Herstellung des Dioxoborolan-Liganden (13):

Zu einer Lösung aus 16,39 g (+)*-N,N,N',N'-*Tetramethyl-L-weinsäurediamid (80,27 mmol, Aldrich batch 11410TA) in 110 ml trockenem Toluol wurden 10,00 g n-Butylboronsäure (98,10 mmol) gegeben und 8 Stunden am Wasserabscheider gekocht. Das Lösungsmittel wurde bei vermindertem Druck abdestilliert und der Rückstand mit 10 ml Dichlormethan versetzt. Man filtrierte von den verbleibenden Kristallen ab, spülte mit 5 ml Dichlormethan und entfernte das Lösungsmittel bei vermindertem Druck am Rotationsverdampfer. Man erhielt 22,0 g von **(13)** als klares, farbloses Öl.

¹H-NMR (400 MHz, CDCl₃): δ = 5.53 (s, 2 H), 3.20 (s, 6 H), 2.98 (s, 6 H) 1.42-1.28 (m, 4 H), 0.89-0.83 (m, 5 H).

¹³C-NMR (100 MHz, CDCl₃): δ = 168.0, 75.56, 37.05, 35.89, 25.82, 25.17, 13.80, 9.99.

### Herstellung des Zn(CH₂l)₂^{·}DME-Komplexes (14) als Lösung in CH₂Cl₂

Unter Schutzgas wurden 37 ml Dichlormethan mit 3,5 g Molsieb vorgelegt. Man kühlte auf -20 °C und versetzte unter Rühren nacheinander mit 18.7 ml Diethylzink (1,0 M in Hexan, 18.7 mmol), 1.95 ml Ethylenglykoldimethylether (DME, 18.7 mmol) und 3.0 ml Diiodmethan (37.5 mmol). Man ließ für 30 min bei -20 °C rühren. Die klare Lösung von **(14)** wurde direkt für die Cyclopropanierung verwendet.

### Enantioselektive Cyclopropanierung von (Z)-2-Decen-1-ol (5):

Unter Schutzgas wurde eine Mischung aus 2,20 g des Dioxoborolan-Liganden (13), 1,17 g 2-Decen-1-ol (5), 3.4 g Molekularsieb 4 Å und 37 ml trockenem Diethylether bereitet und auf -20 °C gekühlt. Mittels einer Teflonkanüle wurde der frisch zubereitete Zₙ(CH₂l₂)^{·}DME-Komplex (14) innerhalb von 2 min bei -20 °C hinzugefügt. Man ließ 2 Stunden bei -10 °C rühren und versetzte dann mit 15 ml ges. Ammoniumchloridlösung. Es wurde vom Feststoff abfiltriert, die organische Phase abgetrennt und die wässrige Phase dreimal mit je 10 ml Diethylether extrahiert. Die vereinigten organischen Phasen wurden 12 h mit 100 ml Natronlauge (5 M) gerührt. Man trennte die org. Phase ab, extrahierte nacheinander mit 20 ml verd. Salzsäure, ges. Natriumhydrogencarbonat- und Kochsalzlösung und trocknete über Natriumsulfat. Nach Abdampfen des Lösungsmittels wurden 3,57 g eines gelben Öls erhalten. Man chromatographierte das Rohprodukt an Kieselgel mit einem Laufmittel Diethylether/Hexan = 1/8. Es wurden 0,8 g eines farblosen Öls von (15) erhalten.

Literatur: A. B. Charette, S. Prescott, C. Brochu, *J*. *Org. Chem.* **1995,** 60, 1081-1083.

### Bestimmung des Enantiomerenüberschusses via Mosher-Ester (17):

Zur Validierung der Methode wurde zunächst das racemische Gemisch des *cis*-2-Heptylcyclopropyl-1-methanols **(6)** umgesetzt. Nachdem sichergestellt war, daß die diastereotopen Protonen 1'-H₂ abgesetzte Signale erzeugen, wurden 33,1 mg des enantiomerenangereicherten Heptylcyclopropyl-1-methanols (15) wie folgt umgesetzt:

33,1 mg des Alkohols **(15)** wurden in 0,5 ml Dichlormethan und 0,36 ml Pyridin gelöst und mit 49,1 mg (R)-(-)-α-Methoxy-α-(trifluormethyl)phenylessigsäurechlorid **(16)** 24 h bei RT gerührt. Die ausgefallenen Salze wurden über Kieselgel abfiltriert und das Lösungsmittel im Vakuum entfernt. Man erhielt jeweils ca. 50 mg eines gelblichen Öls **(17)**. Im ¹H-Spektrum, gemessen bei einer Feldstärke von 400 MHz in CDCl₃, wurden die 1'-H₂-Signale der beiden Diastereomere getrennt voneinander detektiert (Diastereomer 1: 4.49 ppm, dd, *J* = 7.2, 11.4 Hz, 4.15 ppm, dd, J = 8.5, 11.6 Hz; Diastereomer 2: 4.44 ppm, dd, *J* = 7.2, 11.4 Hz, 4.22 ppm, dd, *J* = 8.5, 11.6 Hz). Aus der Integration ergibt sich ein Diastereomerenverhältnis im Bereich 95/5 - 88/12.

### Enantiomeren-angereicherte cis-2-Heptylcyclopropyl-1-carbonsäure (18):

Man löste 1,35 g Chromtrioxid (13,5 mmol) in 9,5 ml Schwefelsäure (2 M, 19 mmol). Unter Rühren wurde bei RT 0,5 g enantiomerenangereichertes (Z)-2-Heptyl-cyclopropyl-1-methanol (15) in 5 ml Aceton zugetropft. Man rühte 38 h bei RT. Es wurden 200 ml Diethylether zugefügt und die Phasen getrennt. Die org. Phase extrahierte man dreimal mit je 50 ml 5 %ige Natronlauge, diese wurde dreimal mit je 100 ml Diethylether gegengeschüttelt. Man säuerte mit 10%iger Salzsäure auf pH = 1 an und trennte die Carbonsäure durch dreimalige Extraktion mit jeweils 100 ml Diethylether ab. Nach Eindampfen am Rotationsverdampfer wurde das Rohprodukt durch Chromatographie an 90 g Kieselgel 60 (230-400 mesh; Hexan/Diethylether =8/1) gereinigt. Man erhielt 0,4 g **(18)** mit ca. 83 % Reinheit (GC). Durch chirale Gaschromatographie an einer GC-Säule IVADEX-3 unter den in Beispiel 6 angegeben Bedingungen wurde ein Enantiomerenüberschuss für das (1 S, 2R) - Enantiomer von ee = 92 % ermittelt.

### Bestimmung der optischen Drehrichtung für die enantiomerenangereicherte cis-2-Heptylcyclopropyl-1-carbonsäure (18):

Die Drehrichtung der optischen Rotation des enantiomerenangereicherten Materials **(18)** wurde mittels Chiralyser bestimmt. Hierzu wurde das Material durch präparative HPLC zunächst weiter aufgereinigt:
- Säule:: Kromasil-100 C18; 5µ; 250x8 mm
- Mobile Phase:: A: Methanol;
- B:: H₂O pH = 3 (Ameisensäure), isokratisch 80% A, 20% B

Nach präparativer HPLC wurde ein Material **(18)** mit 98% Reinheit nach GC erhalten. Nach Kalibrierung der Chiralysers mit (-)-Fructose und (+)-Glucose wurde das aufgereinigte Material **(18)** mit dem Chiralyser vermessen. Die Drehrichtung wurde als (+) bestimmt.

### Beispiel 6: Isolierung der natürlich vorkommenden 2-Heptylcyclopropyl-1-carbonsäure und deren Analyse

Ausgegangen wurde von Orange Residue, der aus Orange Peel Oil gewonnen wird. Der Anteil an Residue in einem Peel Oil liegt meist im Bereich von wenigen Massenprozent, typischerweise im Bereich von 1 bis 5 Gew.-%.

Durch Säure-Base Trennung wurden aus 500 g Orange Residue 1,2 g einer Säurefraktion erhalten. Die Säurefraktion wurde mittels Diazomethan derivatisiert und die resultierenden Methylester mittels HPLC getrennt. Durch diese HPLC-Trennung wurden 65 mg einer Fraktion erhalten, die hauptsächlich Cascarillasäuremethylester enthielt.

Der Gehalt an Cascarillasäure im Orange Residue lag somit bei ca. 130 ppm.

2-Heptylcyclopropyl-1-carbonsäure wurde erstmals sensorisch (per Riech-GC) in einer an Cascarillasäure angereicherten Fraktion detektiert. Ein korrespondierender Peak im GC wurde nicht angezeigt. Durch wiederholte Anreicherung an 2-Heptylcyclopropyl-1-carbonsäure konnte eine Fraktion erhalten werden, welche hauptsächlich aus Cascarillasäure und 2-Heptylcyclopropyl-1-carbonsäure bestand. Das gaschromatographische Verhältnis der Peaks der Cascarillasäure und 2-Heptylcyclopropyl-1-carbonsäure in der mehrfach angereicherten Fraktion lag bei etwa 99 : 1. Insgesamt ergab sich ein Gehalt von 2-Heptylcyclopropyl-1-carbonsäure im Orangen Residue von etwa 1 ppb.

Die Untersuchungen wurden mittels chiraler GC und per Riech-GC (ODP; Olfaktometrie) durchgeführt.

Agilent GC 6890 mit Gerstel KAS 4 Injektor und Agilent 66676 Autosampler
- Säule:: 25m x 0,25mm ID, FT 0,15 mm FT IVADEX-3 (Iva Analysentechnik)
- Temperatur / Ofen:: 80°C mit 2°C/min auf 160 °C
- Trägergas:: He, 1,5 ml/min
- Detektor:: FID (Flammenionisationsdetektor) und ODP (olfaktometrische Bestimmung)
- Probe:: je 1 µl; split 1:20, 0.01 % in Ether

Die folgende absolute Stereochemie konnte den einzelnen GC- Peaks zugeordnet werden:
1. Peak: (1*S*,2*R*)-cis-Isomer, Formel C
2. Peak: (1*R*,2*S*)-cis-Isomer, Formel B
3. Peak: (1*R*,2*R*)-trans-Isomer, Formel D
4. Peak: (1*S,*2*S*)-trans-Isomer, Formel E

Es wurde gefunden, dass die natürlich vorkommende 2-Heptylcyclopropyl-1-carbonsäure Formel C entspricht und die absolute Konfiguration (1*S*, 2*R*) aufweist:

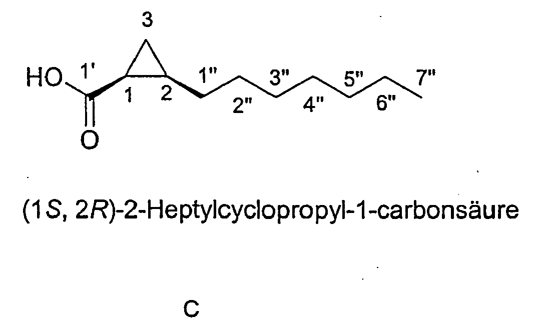

### Beispiel 7: Synthese von Mischungen aus cis-2-Heptylcyclopropyl-1-carbonsäure und trans-2-Heptylcyclopropyl-1-carbonsäure

### 2-Heptylcyclopropyl-1-carbonsäureethylester durch übergangsmetall-katalysierte Reaktion von 1-Nonen mit Diazoessigsäureethylester

### Mit Rhodiumacetat als Katalysator:

In einem 500 ml Dreihalskolben mit gasdichtem Rührer, Innenthermometer und Gasuhr wurden 140,8 g 1-Nonen (1,116 mol) und 456 g Rhodium(II)-acetat dimer (1,03 mmol) vorgelegt. Man heizte die Reaktionsmischung auf 40 °C auf und dosierte über 8 h mit konstanter Geschwindigkeit 50,8 g Diazoessigsäureethylester (445 mmol) zu. Der sofortige Umsatz des zugetropften Diazoessigesters ließ sich durch den Vergleich des gemessenen Gasvolumens mit dem theoretisch Wert an freigesetztem Stickstoff überwachen.

Man filtrierte vom Katalysator ab und gewann das überschüssige 1-Nonen durch Destillation am Rotationsverdampfer bei vermindertem Druck zurück. Das zurückbleibende Öl enthielt laut GC ca. 2,5% Maleinsäureethylester, ca. 2,5% Fumarsäureester, ca. 39% *cis-*Heptylcyclopropyl-1-carbonsäureethylester und ca 56% *trans-*Heptylcyclopropyl-1-carbonsäureester. Der rohe Heptylcyclopropyl-1-carbonsäureethylester ließ sich durch fraktionierende Destillation im Hochvakuum reinigen. Hierbei konnte das *cis-*/*trans*-Verhältnis durch Wahl der entsprechenden Fraktionen eingestellt werden.

### Mit aktivierter Kupferbronze als Katalysator:

In einem 100 ml Dreihalskolben mit Magnetrührer, Rückflußkühler, pneumatischer Wanne mit graduiertem Glasrohr, Innenthermometer und Septum wurde zunächst 1,00 g Kupferbronze vorgelegt und 30 min in 2 ml Eisessig gerührt. Man ließ das Metallpulver absitzen und zog die Essigsäure mit eine Spritzenkanüle vorsichtig ab.

Es wurden 29,0 g 1-Nonen (230 mmol) hinzugefügt und die Reaktionsmischung auf 85 °C erwärmt. Man dosierte über 2 h mit konstanter Geschwindigkeit 2,00 g (17,5 mmol) Diazoessigsäureethylester zu. Der sofortige Umsatz des zugetropften Diazoessigesters ließ sich durch den Vergleich des gemessenen Gasvolumens mit dem theoretisch Wert an freigesetztem Stickstoff überwachen. Laut GC waren im Rohprodukt *cis*-Heptylcyclopropyl-1-carbonsäureethylester und trans- Heptylcyclopropyl-1-carbonsäureester im Verhältnis 37/63 enthalten.

Literatur: L. A. Paquette et a., *J. Am. Chem. Soc*. **1969**, *91,* 7108-7113; P. H. Mazzocchi et al., *J*. *Org. Chem.* **1973,** *38,* 2221-2225.

### 2-Heptylcyclopropyl-1-carbonsäure durch Verseifung von 2-Heptylcyclopropyl-1-carbonsäureethylester

Man legte in einem 500 ml Dreihalskolben eine Lösung aus 31,7 g KOH oder NaOH in 50 ml Wasser vor, verdünnte mit 150 ml Ethanol und versetzte langsam unter Rühren mit 60 g *cis*/*trans*-Heptylcyclopropyl-1-carbonsäureester. Man ließ 18 h bei Raumtemperatur rühren. Durch Verkürzung der Reaktionsdauer kann der *cis*-/*trans-*Anteil der Heptylcyclopropyl-1-carbonsäure eingestellt werden.

Man entfernte am Rotationsverdampfer ca. 100 ml Ethanol und nahm in einer Mischung aus 100 ml Wasser, 50 ml ges. Kochsalzlösung, 300 ml *tert*.Butylmethylester und 20 ml n-Hexan auf. Die wässrige Phase wurde abgetrennt und mit 10%iger HCl auf pH = 1 eingestellt. Man extrahierte dreimal mit je 150 ml *tert*.-Butylmethylester und trocknete die vereinigten organischen Phasen über Natriumsulfat. Nach Entfemen des Lösungsmittels am Rotationsverdampfer wurde an einer 20 cm Vigreux-Kolonne im Vakuum destilliert. Durch Wahl geeigneter Fraktionen läßt sich das *cis*-/*trans*-Verhältnis einstellen.

### Beispiel 8: Synthese von enantiomerenangereicherter cis-2-Heptylcyclopropyl-1-carbonsäure (1R, 2S)

### (2S)-2-Heptylcyclopropryl-1,1-dicarbonsäurediethylester durch Reaktion von Ethylmalonat mit (4R)-4-Heptyl-[1,3,2]dioxathiolan-2,2-dioxid

(4R)-4-Heptyl-[1,3,2]dioxathiolan-2,2-dioxid wurde in drei Stufen nach den Vorschriften von K. B. Sharpless (H. C. Kolb, M. S. VanNieuwenhze, K. B. Sharpless, *Chem. Rev*. **1994,** *94*, 2483-2547; Y. Gao, K. B. Sharpless, *J. Am. Chem. Soc.* **1988,** *110,* 7538-7539) aus 1-Nonen durch cis-Hydroxylierung mit (DHQD)₂PHAL-Liganden (AD-Mix beta), Umsetzung mit Thionylchlorid und anschließende Oxidation zum cyclischen Sulfat unter Rutheniumkatalyse hergestellt.

In einem 50 ml-Zweihalskolben mit Rückflußkühler, Magnetrührer und Septum wurden 0,25 g Natriumhydrid (10,4 mmol) in 20 ml DMF vorgelegt und langsam unter Rühren 0,80 g Malonsäurediethylester (5,00 mmol) zugetropft. Nachdem die Gasentwicklung beendet war, wurde langsam 1,00 g (4*R*)-4-Heptyl-[1,3,2]dioxathiolan-2,2-dioxid (4.50 mmol) hinzugefügt. Man rührte 30 min bei Raumtemperatur und 30 min unter Rückfluß. Man ließ 14 h bei Raumtemperatur nachrühren, versetzte mit 50 ml Wasser und extrahierte dreimal mit 50 ml tert.-Butylmethylether. Die organische Phase wurde mit 30 ml 10%iger HCl-Lösung, 30 ml 10%iger Natriumhydrogencarbonatlösung und 30 ml gesättigter Kochsalzlösung gewaschen, über Natriumsulfat getrocknet und das Lösungsmittel am Rotationsverdampfer entfernt.

Man erhielt 1,32 g eines farblosen Öls mit 87% 2-Heptylcyclopropyl-1,1-dicarbonsäurediethylester nach GC.

Literatur: Y. Gao, K. B. Sharpless, *J. Am. Chem. Soc.* **1988**, *110,* 7538-7539.

### 2-Heptylcyclopropyl-1-carbonsäureethylester durch Decarboxylierung von 2-Heptylcyclopropyl-1,1-dicarbonsäurediethylester mit Lithiumchlorid in DMSO

In einem 25 ml Zweihalskolben wurden 300 mg 2-Heptylcyclopropyl-1,1-dicarbonsäurediethylester (1,05 mmol), 45 mg Lithiumchlorid (1,05 mmol) und 40 mg Wasser (2,22 mmol) in 9 ml DMSO vorgelegt und 8 h unter Rückfluß erhitzt.

Man versetzte mit 10 ml Wasser und extrahierte mit 50 ml *tert.-*Butylmethylether. Die organische Phase wurde mit 10 ml Wasser und 10 ml gesättigter Kochsalzlösung gewaschen, über Natriumsulfat getrocknet und das Lösungsmittel im Vakuum entfernt. Man erhielt 205 mg eines gelblichen Öls. Laut GC waren 17% *cis-* und 21% *trans*-2-Heptylcyclopropyl-1-carbonsäureethylester enthalten.

Literatur: H. Markgraf et al., *J. Org. Chem.* **1977**, 42, 2631-2632.

Bevor das so erhaltene Gemisch aus *cis*- und *trans*-2-Heptylcyclopropyl-1-carbonsäureethylester in die Verseifung eingesetzt wurde, erfolgte eine weitere chromatographische Aufreinigung an Ag-dotiertem Kieselgel.

### 2-Heptylcyclopropyl-1-carbonsäure durch Verseifung von 2-Heptylcyclopropyl-1-carbonsäureethylester

Die Verseifung erfolgte wie in Beispiel 7 beschrieben.

Durch chirale Gaschromatographie an einer GC-Säule IVADEX-3 unter den in Beispiel 6 angegeben Bedingungen wurde ein Enantiomerenüberschuss für das (1 R, 2S) - Enantiomer von ee = 77% ermittelt. Dieses nicht natürliche cis-Enantiomer der Formel B zeigt einen sehr viel schwächeren Geruch als das natürlich vorkommende (1 S, 2R) - Enantiomer der Formel C.

### Beispiel 8: Anwendunasbeispiele:

### Beispiel 8.1:

Natürliche (1S, 2R) 2-Heptylcyclopropyl-1-carbonsäure (Formel C) wurde in Kombination mit ausgewählten Citrus-Geschmackstoffen in einer Konzentration von 5 bis 10 ppb einem Getränk zugesetzt. Im Vergleich mit dem Getränk ohne Zusatz wurde das Getränk mit Zusatz sensorisch wie folgt bewertet: "more body, more fruity, more impact" (voller, fruchtiger, stärker). Stabititätstests zeigten zudem eine Verbesserung hinsichtlich der Geschmacksretention.

### Beispiel 8.2:

Auch Kombinationen von (1*S*, 2*R*) 2-Heptylcyclopropyl-1-carbonsäure (Formel C) mit (Z)-8-Tetradecenal und 4,5-Epoxy-(E)-2-Decenal zeigten eine verbesserte geschmackliche Leistung.

## Patentansprüche

1. Gegebenenfalls isoliertes und/oder aufgereinigtes
Enantiomer der 2-Heptylcyclopropyl-1-carbonsäure
oder
Gemisch von zwei, drei oder sämtlichen Enantiomeren der 2-Heptylcyclopropyl-1-carbonsäure.

2. Gemisch beider Enantiomeren
der cis-2-Heptylcyclopropyl-1-carbonsäure oder
der trans-2-Heptylcyclopropyl-1-carbonsäure.

3. Gemisch nach Anspruch 2, wobei ein Enantiomerenüberschuss von zumindest 40 % eingestellt ist.

4. Duftstoff- und/oder Aromastoffkomposition, umfassend eine sensorisch wirksame Menge eines gegebenenfalls isolierten und/oder aufgereinigten Enantiomeren oder eines Gemisches von Enantiomeren nach einem der Ansprüche 1-3.

5. Duftstoff- und/oder Aromastoffkomposition nacht Anspruch 4 umfassend einen Anteil von zumindest 0,001 Massenprozent, vorzugsweise 0,01 Massenprozent, besonders vorzugsweise 0,1 Massenprozent des Enantiomeren bzw. des Gemisches von Enantiomeren, bezogen auf die Gesamtmasse der Duftstoff- oder Aromastoffkomposition.

6. Aromatisiertes Lebensmittel, umfassend eine sensorisch wirksame Menge eines Enantiomeren oder eines Gemisches von Enantiomeren nach einem der Ansprüche 1-3.

7. Körperpflegeprodukt, Reinigungsmittel oder sonstiges nicht zum Verzehr bestimmtes Produkt, umfassend eine sensorisch wirksame Menge eines Enantiomeren oder eines Gemisches von Enantiomeren nach einem der Ansprüche 1-3.

8. Verwendung eines Enantiomeren oder eines Gemisches von Enantiomeren nach einem der Ansprüche 1-3 als Duft- und/oder Aromastoff.

9. Verfahren zum Erzeugen oder Modifizieren einer Duftstoff oder Aromastoffkomposition, mit folgenden Schritten:
- Bereitstellen einer Ausgangskomposition und
- Vermischen der Ausgangskomposition mit einer sensorisch wirksamen Menge eines Enantiomeren oder eines Gemisches von Enantiomeren nach einem der Ansprüche 1-3.

10. Verfahren zum Modifizieren einer Aromastoffkomposition in Richtung frischer, fruchtiger, voller und/oder schaliger, wobei die Aromastoffkomposition mit einer sensorisch wirksamen Menge eines Enantiomeren nach Anspruch 1 oder eines Gemisches beider Enantiomeren der cis-2-Heptylcyclopropyl-1-carbonsäure nach Anspruch 2 vermischt wird.

11. Verfahren zur selektiven Herstellung von cis- oder trans-2-Heptylcyclopropyl-1-carbonsäure nach Anspruch 1 wobei zur Einstellung der *cis-* bzw. trans-Konfiguration der Substituenten am Cyclopropan-Ring von einem Precursor mit einer Z- bzw. E-konfigurierten Doppelbindung ausgegangen und diese selektiv cyclopropaniert wird.

## Claims

1. Optionally isolated and/or purified enantiomer of 2-heptylcyclopropane-1-carboxylic acid or a mixture of two, three or all of the enantiomers of 2-heptylcyclopropane-1-carboxylic acid.

2. Mixture of the two enantiomers of cis-2-heptylcyclopropane-1-carboxylic acid or of trans-2-heptylcyclopropane-1-carboxylic acid.

3. Mixture according to Claim 2 wherein an enantiomeric excess of at least 40% is created.

4. Perfume and/or flavouring composition comprising a sensorially effective amount of an optionally isolated and/or purified enantiomer or of a mixture of enantiomers according to one of Claims 1-3.

5. Perfume and/or flavouring composition according to Claim 4 comprising a proportion of at least 0.001 percent by weight, preferably of 0.01 percent by weight and particularly preferably of 0.1 percent by weight of the enantiomer or enantiomer mixture, based on the total weight of the perfume or flavouring composition.

6. Flavoured food comprising a sensorially effective amount of an enantiomer or enantiomer mixture according to one of Claims 1-3.

7. Body care product, cleaning agent or other product not intended for consumption comprising a sensorially effective amount of an enantiomer or enantiomer mixture according to one of Claims 1-3.

8. Use of an enantiomer or enantiomer mixture according to one of Claims 1-3 as a perfume and/or flavouring.

9. Process for the production or modification of a perfume or flavouring composition, comprising the following steps:
- preparation of a starting composition and
- mixing of the starting composition with a sensorially effective amount of an enantiomer or enantiomer mixture according to one of Claims 1-3.

10. Process for the modification of a flavouring composition in a fresher, fruitier, fuller and/or more peel-like direction, wherein the flavouring composition is mixed with a sensorially effective amount of an enantiomer according to Claim 1 or of a mixture of the two enantiomers of cis-2-heptylcyclopropane-1-carboxylic acid according to Claim 2.

11. Process for the selective preparation of cis- or trans-2-heptylcyclopropane-1-carboxylic acid according to Claim, wherein, to establish the cis or trans configuration of the substituents on the cyclopropane ring, a precursor with a double bond of Z or E configuration is used as the starting material and this double bond is selectively cyclopropanated.

## Revendications

1. Enantiomère éventuellement isolé et/ou purifié de l'acide 2-heptylcyclopropyl-1-carboxylique ou mélange de deux, trois ou tous les énantiomères de l'acide 2-heptylcyclopropyl-1-carboxylique.

2. Mélange des deux énantiomères de l'acide cis-2-heptylcyclopropyl-1-carboxylique ou de l'acide trans-2-heptylcyclopropyl-1-carboxylique.

3. Mélange selon la revendication 2 où un excès énantiomérique d'au moins 40 % est établi.

4. Composition de parfum et/ou d'arôme comprenant une quantité efficace du point de vue sensoriel d'un énantiomère éventuellement isolé et/ou purifié ou d'un mélange d'énantiomères selon l'une des revendications 1-3.

5. Composition de parfum et/ou d'arôme selon la revendication 4 comprenant une proportion d'au moins 0,001 % en masse, de préférence de 0,01 % en masse, de manière pratiquement préférable de 0,1 % en masse de l'énantiomère ou du mélange d'énantiomères par rapport à la masse totale de la composition de parfum ou d'arôme.

6. Aliment aromatisé comprenant une quantité efficace du point de vue sensoriel d'un énantiomère ou d'un mélange d'énantiomères selon l'une des revendications 1-3.

7. Produit pour les soins corporels, produit de nettoyage ou autre produit non destiné à la consommation comprenant une quantité efficace du point de vue sensoriel d'un énantiomère ou d'un mélange d'énantiomères selon l'une des revendications 1-3.

8. Utilisation d'un énantiomère ou d'un mélange d'énantiomères selon l'une des revendications 1-3 comme parfum et/ou arôme.

9. Procédé pour produire ou modifier une composition de parfum ou d'arôme comportant les étapes suivantes :
- préparation d'une composition initiale et
- mélange de la composition initiale avec une quantité efficace du point de vue sensoriel d'un énantiomère ou d'un mélange d'énantiomères selon l'une des revendications 1-3.

10. Procédé pour modifier une composition d'arôme dans le sens frais, fruité, riche et/ou discret, où la composition d'arôme est mélangée avec une quantité efficace du point de vue sensoriel d'un énantiomère selon la revendication 1 ou d'un mélange des deux énantiomères de l'acide cis-2-heptylcyclopropyl-1-carboxylique selon la revendication 2.

11. procédé pour la production sélective d'acide cis- ou trans-2-heptylcyclopropyl-1-carboxylique selon la revendication 1 où, pour l'établissement de la configuration cis- ou trans- des substituants sur le cycle cyclopropane, on part d'un précurseur ayant une double liaison de configuration Z ou E que l'on cyclopropane sélectivement.
